# EUROPÄISCHE PATENTSCHRIFT

(11) **EP 2 818 152 B1**
(45) Veröffentlichungstag und Bekanntmachung des Hinweises auf die Patenterteilung: **09.08.2017**
(21) Anmeldenummer: 14172543.2
(22) Anmeldetag: 16.06.2014
(51) Int. Cl.: A61J 1/14, A61M 39/10, A61M 39/18

(54) **Luer-Lock-Verbinder mit Nuten**
Luer-lock connector with grooves
Raccord Luer-Lock rainuré

(30) Priorität: 24.06.2013 DE 102013106550
(43) Veröffentlichungstag der Anmeldung: 31.12.2014
(73) Patentinhaber: B. Braun Avitum AG, 34212 Melsungen (DE)
(72) Erfinder: Wesseler, Matthias, 49326 Melle (DE)
(74) Vertreter: Winter, Brandl, Fürniss, Hübner, Röss, Kaiser, Polte - Partnerschaft mbB

(56) Entgegenhaltungen:
- EP-A1- 1 859 773
- WO-A2-2007/149960
- DE-A1-102008 048 988
- DE-C1- 3 926 395
- DE-C1- 4 313 636

## Beschreibung

### Luer-Lock-Verbinder mit Nuten

Die vorliegende Erfindung betrifft ein Konnektorsystem oder Verbindungssystem für einen Flüssigkeitsbehälter für pharmazeutische Lösungen, mit einem in ein Fluidleitsystem wie einen Schlauch oder eine Beuteltülle einsteckbaren Außenteil und mit einem darin angeordneten, vom Außenteil stofflich separaten Innenteil, wobei an dem Außenteil ein an einer Sollbruchstelle angebundener und mit Handkraft abreißbarer Originalitätsverschluss vorhanden ist, wobei ferner das Innenteil mit seiner Außenseite zumindest abschnittsweise an einer Innenseite des Außenteils anliegt, und ein Fluiddurchfluss durch das Innenteil zusätzlich zum Originalitätsverschluss mittels eines am Innenteil angebrachten Verschlusselements verhindert ist. Die vorliegende Erfindung betrifft auch ein medizinisches Versorgungssystem, mit einem Beutel wie einem Dialysebeutel, in dem ein erfindungsgemäßes Konnektorsystem integriert oder eingesetzt ist.

### Hintergrund der Erfindung

In der Medizintechnik, doch auch auf anderen Gebieten wie z. B. im Laborbereich, ist es oftmals erforderlich, Flüssigkeit in einer bestimmten Reihenfolge von einem Behälter in einen anderen zu überführen und sie nach einer bestimmten Zeit oder einem vorgegebenen Ablauf, etwa einem Mischen oder einer chemischen Reaktion, weiterzuleiten. Als Beispiel sei die Dialyse nach dem Hämofiltrationsverfahren genannt. Dort kommt beispielsweise eine Bicarbonat-Elektrolytlösung als Substituat zur Anwendung.

Dialysebeutelanschlüsse sollen dabei üblicherweise vorgehalten werden, um einen effizienten Verschluss bei gleichzeitig leichter Öffenbarkeit zu gewährleisten.

### Stand der Technik

Aus dem Stand der Technik, etwa der DE 43 13 636 C1 ist ein Konnektorsystem zum Verbinden von Flüssigkeitsbehältern bekannt. Dort ist beispielsweise ein Konnektorsystem zum Verbinden von Flüssigkeitsbehältern mit medizinischen Flüssigkeiten sowie einem der Flüssigkeitsbehälter mit einem Überleitungsset oder einem weiteren Behälter bekannt, wobei das Konnektorsystem zum Einhalten einer vorgegebenen Verbindungsfolge ein erstes und ein zweites Verbindungsteil mit jeweils einem ersten Anschluss verbindet. Die Anschlüsse sind zum Verbinden mit entsprechenden weiteren Anschlüssen an bestimmten Flüssigkeitsbehältern ausgebildet. Ein Verbinden der beiden Verbindungsteile ist miteinander ermöglicht. Ein Zwischenteil mit zwei miteinander verbundenen Anschlüssen, von denen der erste zum lösbaren Verbinden mit dem zweiten Anschluss des ersten Verbindungsteils sowie mit einem Anschlussstück des Überleitungssets und der zweite zum unlösbaren Verbinden mit dem zweiten Anschluss des zweiten Verbindungsteils ausgebildet ist, ist dabei offenbart. Das Zwischenteil vor Herstellung der Verbindung mit dem zweiten Verbindungsteil ist im Inneren des ersten Verbindungsteils angeordnet.

Ein ähnlicher Stand der Technik ist auch aus der DE 41 14 246 C2 bekannt. Dort wird ein Konnektor für einen Behälter für pharmazeutische Lösungen vorgestellt, der mit einem schlauchförmigen Anschlussteil versehen ist, umfassend ein in das Anschlussteil einsteckbaren ersten Kopplungsteil mit einem ersten Durchflusskanal und einem in dieses einsteckbaren zweiten Kopplungsteil mit einem zweiten Durchflusskanal, das mit einer Schlauchleitung verbunden ist, und umfassend ein Absperrorgan. Dabei ist bemerkenswert, dass das erste Kopplungsteil an der Einstecköffnung mit einem gummielastischen Stopfen abgeschlossen ist, über dem sich eine dichtende Verschlusskappe befindet, die mit einem deren zentrale Öffnung abschließenden Abbrechverschluss versehen ist, und die zentrale Öffnung Arretierungsvorsprünge für das in diese einsteckbare zweite Kopplungsteil aufweist, an dem sich mit den Arretierungsvorsprüngen korrespondierende Vorsprünge befinden.

Benachbarter Stand der Technik ist auch aus den Druckschriften DE 10 2008 048 988 A1, WO 2007/149960 A2, EP 1 859 773 A1 und DE 39 26 395 C1 bekannt.

Aus dem Stand der Technik sind ferner Verschlusselemente nach Art von Brechkreuzen bekannt, wobei solche Infusionsbeutel aus Soft-PP-Verbundfolie hergestellt sind.

Es soll ein Dialysebeutelanschluss mit einem Brechkreuz / Abbrechkreuz und einem Originalitätsverschluss vorgesehen werden, wobei das Brechkreuz seinerseits als Originalitätsverschluss dient und in Beutelrichtung eingesetzt ist sowie zum sterildichten Verschluss eines Luer-Innenkegels durch einen weiteren Abbrech-(Abdreh-) Originalitätsverschluss ergänzt wird.

Bisher wurde ein Luer-Anschlussbereich üblicherweise mit einer Schraubkappe verschlossen. Diese hat jedoch den Nachteil, dass sie bei einem Heißdampfsterilisieren nicht vollständig dicht abschließt. Es ist die Folge, dass sich der Anschluss-Luer-Innenkegel mit Kondensat bzw. Wasser aus dem Sterilisationsprozess füllt. Dies soll aber vermieden werden.

Ferner soll ein Schlauchkonnektor mit beutelseitigem Brechkreuz als Sterilitätsverschluss geschaffen werden, bei dem der zur Benutzerseite weisende Innenraum des Luer-Kegels sterildicht von der Außenatmosphäre abgeschlossen ist und bei dem der eingeschlossene Raum sterilisierbar ist.

### Kurzbeschreibung der Erfindung

Diese Aufgabe wird erfindungsgemäß dadurch gelöst, dass an dem Innenteil und dem Außenteil wenigstens ein sich über eine gewisse Länge erstreckendes Fluidleitelement wie eine Rille, eine Riefe, eine Furche oder eine Nut vorhanden ist, wobei das Fluidleitelement (15) derart dimensioniert ist, dass es kapillarartig wirkt, sodass ein Sterilisationsmittel, wie Wasserdampf, zwischen das Innenteil (10) und das Außenteil (4) geleitet werden kann und einen Innenraum (18) im Inneren des Innenteils (10) erreicht, aber das Fluidleitelement (15) so bemessen ist, dass es ein Tropfen ausschließt. Unter einem Fluidleitelement wird somit in allgemeiner Form eine eine Vertiefung schaffende Ausprägung verstanden, die rillen-, riefen-, furchen- oder nutartig ausgebildet sein kann. Durch die Dimensionierung des Fluidleitelements derart, dass es kapillarartig wirkt, kann Sterilisationsmittel wie Dampf auch zwischen das Innenteil und das Außenteil geleitet werden, insbesondere in einen Konnektionsraum im Inneren des Innenteils.

Dies ist insbesondere für die Montage vorteilhaft, weil dann Luft entweichen kann. Die Fluidleitelemente sind so bemessen, dass sie ein Tropfen ausschließen, aber nicht sterildicht sind. Ferner sichern Sie das Innenteil zum Außenteil gegen Rotation.

Es wird daher ein Luer-Lock-Verbinder geschaffen, der für Schlauchsysteme geeignet ist und bei mit Originalitätsverschluss versehenen oder zu versehenden flüssigkeitsgefüllten Beuteln einsetzbar ist. Ein Innenraum darin ist dann sterilisierbar. In den Innenraum kann über kapillarartige Nuten, also Ausprägungen der Fluidleitelemente, Dampf eingeleitet werden. Über ein in Richtung des Beutels zeigendes Verschlusselement, nach Art eines Brechkreuzverschlusses, kann die freie Entnahme des Inhalts erreicht werden.

Das Innenteil, das auch als Unterteil bezeichnet werden kann, ist auf seiner äußeren Mantelfläche mit einer oder mehreren axial verlaufenden kapillarartigen Nuten versehen. Diese Nuten sind derart ausgebildet, dass das Innenteil im zusammengesteckten Zustand mit dem Außenteil, das auch als Oberteil bezeichnet werden kann, eine kapillare Verbindung aufweist / ermöglicht, die das Eindringen von Wasserdampf, Luft oder Flüssigkeit in den Innenbereich des montierten Verschlusses zulässt.

Zum einen ist, wie bereits erwähnt, hierdurch ein Entweichen der Luft aus dem Innenraum des Bauteils während der Montage gewährleistet, zum anderen kann während der Dampfsterilisation Wasserdampf in den Innenraum strömen und so eine ausreichende Sterilisation sichergestellt werden.

Mit anderen Worten kann der Konnektorinnenraum somit so abgeschlossen werden, dass der Zwischenraum zwischen dem Innen- und Außenteil in den Sterilisationsprozess einbezogen werden kann. Es kann ein sterildichter Verschluss des Luer-Innenkegels erreicht werden und ein Brechkreuz zur Freigabe des Durchflusses genutzt werden.

Dadurch wird ein Luer-Lock-Anschluss mit sterildichtem Originalitätsverschluss des Luer-Innenkegels, sterildichtem Innenraum des gesamten Konnektors und Brechkreuzverschluss zur Freigabe eines Medienstroms realisiert.

Vorteilhafte Ausführungsformen sind auch in den Unteransprüchen beansprucht und werden nachfolgend näher erläutert.

So ist es ein Vorteil, wenn eine Vielzahl von Fluidleitelementen vorhanden ist, vorzugsweise vier, fünf, sechs, sieben, acht, neun, zehn, elf, zwölf oder mehr Fluidleitelemente. Allerdings sind auch zwei oder drei Fluidleitelemente einsetzbar. Auf diese Weise kann eine gleichmäßige Durchdringung des Spaltbereichs zwischen dem Innenteil und dem Außenteil bewirkt werden, was zu einer guten Sterilisation führt.

Für die Sterilisationswirkung ist es von Vorteil, wenn die Fluidleitelemente gleichverteilt über den Umfang des Innenteils angeordnet sind und geradlinig oder kurvig verlaufen. Natürlich können die Fluidleitelemente auch gleichverteilt über den Innenumfang des Außenteils angeordnet sein und auch dort geradlinig oder kurvig verlaufen. Für eine geradlinige Ausprägung ist eine einfache Herstellbarkeit anzuführen, wohingegen eine kurvige, insbesondere gewindeartige oder spiralförmige Ausprägung der Fluidleitelemente andere Vorteile aufweist.

Ein vorteilhaftes Ausführungsbeispiel ist auch dadurch gekennzeichnet, dass Fluidleitelemente in die Außenmantelfläche/Außenoberfläche des Innenteils und/oder die Innenmantelfläche/Innenoberfläche des Außenteils eingearbeitet sind. Es ist sogar möglich, dass ein Fluidleitelement in die Außenmantelfläche, d. h. die Außenoberfläche des Innenteils, und ein anderes Fluidleitelement in die Innenmantelfläche, d. h. die Innenoberfläche des Außenteils, eingearbeitet ist und sich die Fluidleitelemente über den Umfang gesehen entsprechend verteilen.

Als zweckmäßig hat es sich herausgestellt, wenn das Außenteil eine von einem Zylinder bestimmte Innenmantelfläche aufweist und/oder das Innenteil eine von einem Zylinder bestimmte Außenmantelfläche aufweist. Das Innen- bzw. Außenteil kann dann hohlzylinderartig ausgestaltet werden, wobei jedoch nur eine der beiden Flächen eines der beiden Teile zylinderartig ausgeführt ist.

Es ist möglich, dass die Fluidleitelemente sich von einem dem Originalitätsverschluss abgewandten Ende des Außenteils bis zu einem dem Originalitätsverschluss zugewandten Ende des Außenteils derart erstrecken, dass Flüssigkeit auf die Innenseite des Originalitätsverschlusses und/oder des Verschlusselements gelangt. So kann bei Nutzen der Kapillarwirkung Sterilisationsmittel auch in dem Bereich zwischen dem Originalitätsverschluss und dem Verschlusselement, ergo dem Inneren des Innenteils / Kombination aus Innenraum im Originalitätsverschlus und dem Innenraum des Innenteils (d.h. dem Konnektionsraum), wirken.

Für eine Kompatibilität mit anderen Systemelementen ist es von Vorteil, wenn das Außenteil auf seiner Außenseite und/oder das Innenteil auf seiner Innenseite nach Art eines Luer-Konus ausgeformt ist. Auch ist es von Vorteil, wenn auf der dem Originalitätsverschluss zugewandten Seite des Außenteils ein Luer-Lock-Außengewinde vorhanden ist oder ein Luer-Slip-Fortsatz vorhanden ist.

Wenn der Originalitätsverschluss als flügelförmige Handhabe ausgebildet ist, so kann mit einfachen Mitteln, etwa unter Nutzung der humanen Handkraft, das Konnektorsystem auf einer Seite geöffnet werden. Alternativ oder zusätzlich von Vorteil ist es, wenn das Verschlusselement als an einer Sollbruchstelle angebundenes Brechkreuz ausgeformt ist. Solche Brechkreuze können auch durch einen Beutelanschluss oder einen Schlauch hindurch durch normale Fingerkraft betätigt werden und zu einer Öffnung des Konnektorsystems auf der anderen Seite führen.

Für die Erreichbarkeit des Brechkreuzes ist es von Vorteil, wenn das Brechkreuz an einem dem Originalitätsverschluss abgewandten Ende des Innenteils angebunden ist.

Es ist auch von Vorteil, wenn das Innenteil am Außenteil über eine form-, kraft- und/oder stoffschlüssige Verbindung, wie eine Verschweißung, Verklebung, Verpressung, Verschnappung, etwa mittels eines Hinterschnitts und/oder einer umlaufenden Feder gesichert ist.

Ein vorteilhaftes Ausführungsbeispiel ist auch dadurch gekennzeichnet, dass eine durch das Fluidleitelement gebildete Vertiefung eine in Radialrichtung gemessene Tiefe hat, die kleiner als eine Dicke der Wandung des Außenteils und/oder des Innenteils ist.

Dabei kann ein solches Ausführungsbeispiel vorteilhaft weitergebildet werden, wenn die Tiefe über die Länge des Fluidleitelements gleich bleibt oder aber kontinuierlich oder sprunghaft variiert. Ändert sich beispielsweise die Tiefe kontinuierlich nach Art einer progressiven oder degressiven Kurve, so können bestimmte vorteilhafte Effekte erzielt werden.

Ein vorteilhaftes Ausführungsbeispiel ist auch dadurch gekennzeichnet, dass die Tiefe zu klein ist, um zu einer kompletten Perforation des Innenteils oder des Außenteils zu führen. Die Integrität des Konnektorsystems wird dadurch nicht gefährdet.

Auch ist es von Vorteil, wenn das Innenteil in das Außenteil dichtend eingepresst ist. Dies ist eine besonders einfache Art der Verbindung des Innenteils mit dem Außenteil und aus Kosteneinspargründen favorisiert.

Es wird somit ein Schlauchverschluss möglich, bestehend aus einem teilweise zylindrischen Innenteil, welches innen über einen Luer-Hohlkegel verfügt und einseitig offen ist und an dem geschlossenen Ende mit einem Brechkreuz zur Öffnung des Durchgangs verschlossen ist. Dieses zylindrische Innenteil ist dichtend in ein korrespondierendes Außenteil eingepresst. Das Außenteil ist innen zylindrisch zur Aufnahme des oben beschriebenen Innenteils ausgebildet. Weiterhin trägt das Außenteil ein Außengewinde gemäß der Luer-Norm.

Oberhalb der Gewindegänge ist eine Sollbruchstelle in Form einer dünnen umlaufenden Wand angeordnet. Oberhalb dieser Sollbruchstelle befindet sich eine flügelförmige Handhabe, die zum Abscheren der Sollbruchstelle durch eine Drehbewegung dient und dadurch den Luer-Innenkegel freilegt.

Diese zusammengefügte Einheit aus Innen- und Außenteil kann z. B. vorab durch hochenergetische Strahlung (z. B. Gammastrahlung) oder im eingebauten Zustand durch Heißdampfsterilisation keimfrei gemacht werden. Zwecks einer Heißdampfsterilisation kann die Innenwand des Außenteils oder die Außenwand des Innenteils mit achsparallel verlaufenden Riefen versehen sein, so dass die im Beutel befindliche Flüssigkeit bzw. deren Dampf die Zwischenräume zwischen Innen- und Außenteil erreichen kann.

Eine feste Verbindung zwischen Innen- und Außenteil kann z. B. durch Verpressen, Verschnappen, beispielsweise unter Nutzung eines Hinterschnitts und einer umlaufenden Feder, Verschweißen, Verkleben oder sonstigen Verbindungsarten erfolgen.

Der Bedienungsablauf ist dadurch gekennzeichnet, dass bei dem in den Beutelschlauchanschluss eingepressten Luer-Konnektor an seinem Außenende durch Abdrehen des Originalitätsverschlusses der Luer-Innenkegel freigelegt wird. Ein Verbraucher wird über einen männlichen Luer-Lock-Anschluss (Male-Luer-Lock-Anschluss) durch Verschrauben konnektiert. Das Brechkreuz im Beutelschlauch wird zu gegebenem Zeitpunkt abgebrochen. Der Inhalt des Beutels fließt dann zum Verbraucher.

### Figurenbeschreibung

Die Erfindung wird nachstehend anhand einiger bevorzugter Ausführungsbeispiele unter Bezugnahme auf die begleitenden Figuren näher erläutert. Es zeigen:
- Figur 1: ein erfindungsgemäßes Konnektorsystem im Längsschnitt,
- Figur 2: eine Variante eines zusammengebauten Konnektorsystems,
- Figur 3: eine Vergrößerung des Bereichs III aus Figur 2,
- Figur 4: die noch von einander getrennten Innen- und Außenteile des Konnektorsystems in einem in ein Fluidsystem uneingesetzten Zustand,
- Figur 5: eine Variante eines Außenteils für ein erfindungsgemäßes Konnektorsystem,
- Figur 6: eine Vergrößerung eines der Figur 3 entsprechenden Bereichs eines Konnektorsystems, wobei der Längsschnitt im Bereich der Riefen, die die Fluidleitelemente ausbilden, gelegt ist und wobei dieser Bereich als VI in Figur 7 im Gesamtkontext dargestellt ist,
- Figur 7: ein Konnektorsystem nach Art der Erfindung in einer Längsschnittdarstellung durch die Fluidleitelemente,
- Figur 8: eine Vergrößerung des Bereichs VIII aus Figur 7,
- Figur 9: einen Querschnitt quer zur Längsachse des Konnektorsystems aus Figur 7,
- Figur 10: eine Längsschnittdarstellung nur des Außenteils des Konnektorsystems aus Figur 7,
- Figur 11: eine Variante zum Außenteil des Konnektorsystems aus Figur 7,
- Figur 12: das Innenteil des Konnektorsystems aus Figur 7, und
- Figur 13: eine schematische Darstellung des Fluidflusses durch ein erfindungsgemäßes Konnektorsystem.

Die Figuren sind lediglich schematischer Natur und dienen nur dem Verständnis der Erfindung. Die gleichen Elemente sind mit denselben Bezugszeichen versehen.

Die einzelnen Merkmale der Ausführungsbeispiele können auch untereinander kombiniert werden und/oder miteinander ausgetauscht werden.

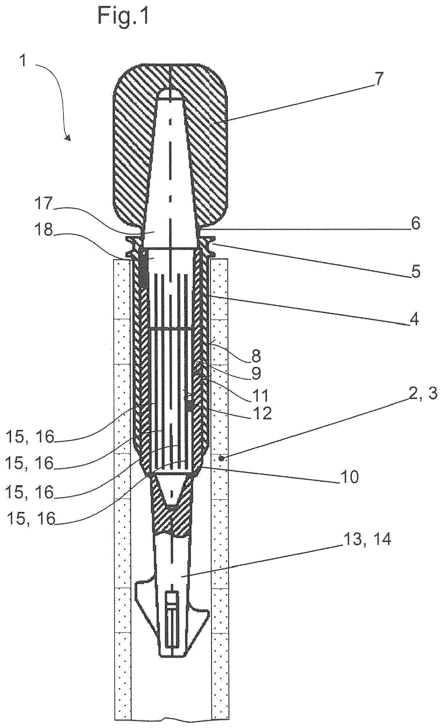

In Figur 1 ist eine erste Ausführungsform eines erfindungsgemäßen Konnektorsystems 1 wiedergegeben.

Das Konnektorsystem ist zum Einsatz an einem Flüssigkeitsbehälter vorgesehen, wobei in dem Flüssigkeitsbehälter pharmazeutische Lösungen aufbewahrbar sind. Das Konnektorsystem 1 ist in ein Fluidleitsystem 2 eingesteckt. Das Fluidleitsystem 2 ist als Schlauch 3 ausgebildet. Es kann aber auch als Schlauchabschnitt oder Beuteltülle ausgebildet sein.

Das Konnektorsystem 1 weist ein Außenteil 4 auf und hat eine Außenform nach der wohlbekannten Luer-Norm. Es kann an einem mittigen oder endnahen Bereich ein Luer-Lock-Außengewinde 5 vorhanden sein. An das Luer-Lock-Außengewinde 5 schließt sich eine Sollbruchstelle 6 an, die es ermöglicht, dass eine dort angebundene Ausprägung eines Originalitätsverschlusses 7, nach Art einer flügelförmigen Handhabe, vom Außenteil 4 abreißbar ist. Das Außenteil 4 weist eine Außenseite 8 und eine Innenseite 9 auf. Im Außenteil 4 steckt auch mittels eines Presssitzes gesichert ein Innenteil 10, das ebenfalls eine Außenseite 11 und eine Innenseite 12 aufweist. Die Außenseite 8 des Außenteils 4 kann auch als Außenmantelfläche oder Außenoberfläche bezeichnet werden. Die Innenseite 9 des Außenteils kann auch als Innenmantelfläche oder Innenoberfläche bezeichnet werden. Die Außenseite 11 des Innenteils 10 kann ebenfalls als Außenmantelfläche oder Außenoberfläche bezeichnet werden. Die Innenseite 12 des Innenteils 10 kann auch als Innenmantelfläche oder Innenoberfläche bezeichnet werden.

Auf der dem Originalitätsverschluss 7 abgewandten Seite bzw. am anderen Ende des Konnektorsystems 1 ist ein Verschlusselement 13 vorhanden. Das Verschlusselement 13 ist als Brechkreuz 14 ausgebildet.

Das Innenteil 10 steckt konzentrisch im Außenteil 4.

Das Außenteil 4 weist auf seiner Außenseite 8 einen Luer-Konus auf. Das Innenteil 10 weist auf seiner Innenseite 12 ebenfalls einen Luer-Konus auf, also einen Luer-Innenkegel.

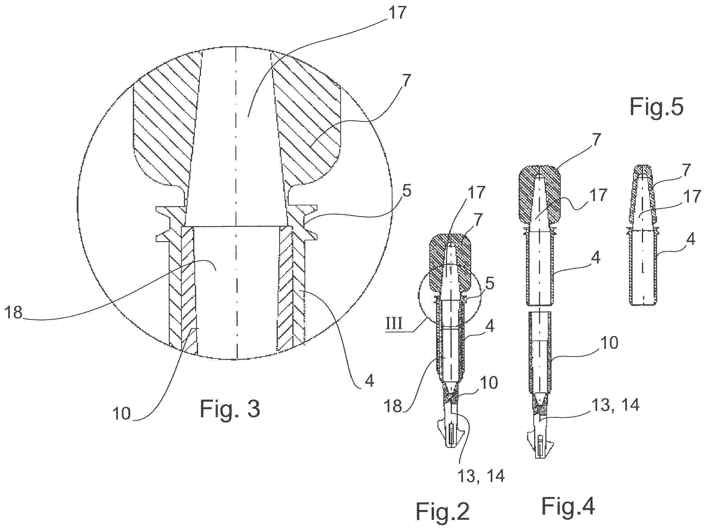

Zwischen dem Innenteil 10 und dem Außenteil 4 sind Fluidleitelemente 15 vorhanden. Die Fluidleitelemente 15 sind nach Art von Längsriefen 16 ausgestaltet. In Figur 2 ist der Zusammenbau des Innenteils 10 und des Außenteils 4 wiedergegeben. Der Bereich des Luer-Lock-Außengewindes 5 ist in Figur 3 größer dargestellt. Dabei ist auch ein Innenraum 17 im Originalitätsverschluss 7 gut zu erkennen. Dieser Innenraum 17 geht in einen Innenraum 18 des Innenteils 10 über. Während in Figur 4 noch die beiden unzusammengesetzten Innen- und Außenteile 10 und 4 dargestellt sind, ist in Figur 5 nur das Außenteil 4 mit abgewandelter Originalitätsverschlussform wiedergegeben. Die Flügel fallen hier kleiner aus. Allerdings zeigt sich ein solcher Längsschnitt auch, wenn nur von zwei Seiten eines Originalitätsverschlusses 7 abstehende Flügel vorhanden sind, welche in derselben Ebene befindlich sind, und der Längsschnitt in einer dazu quer, vorzugsweise orthogonal ausgerichteten Stellung genutzt ist.

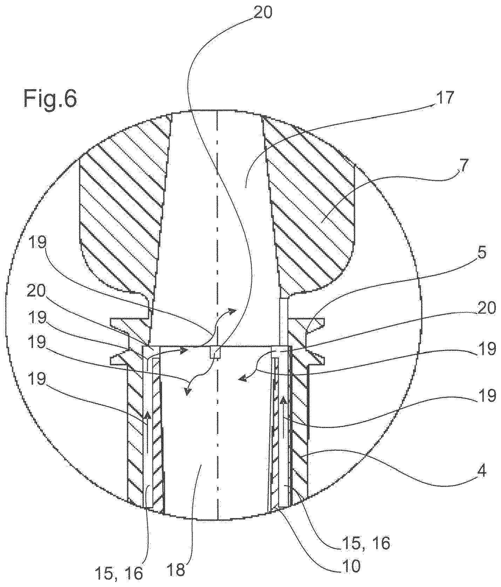

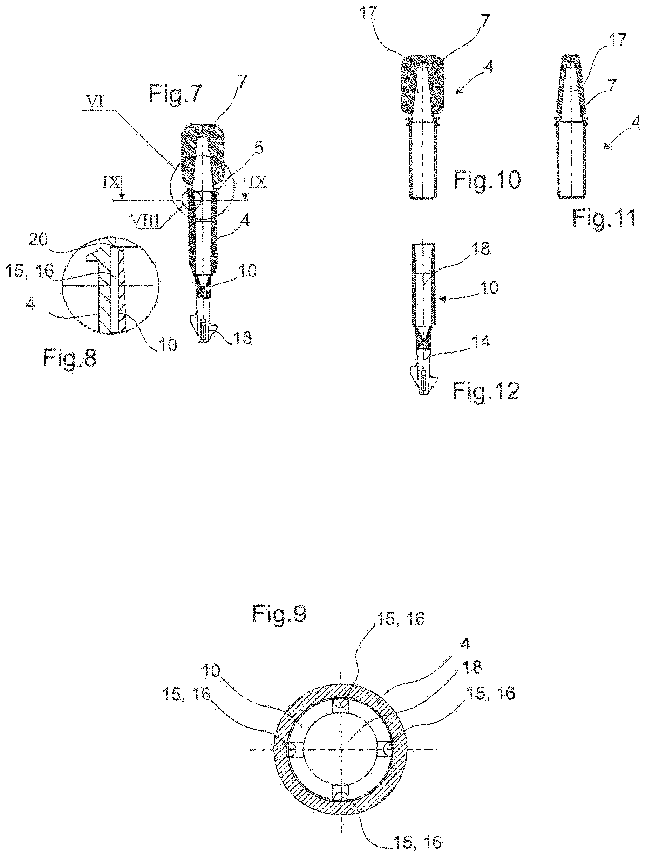

Die Figuren 6 bis 12 machen das räumliche Verstehen der Ausrichtung des Fluidleitelements 15 einfacher. So ist gut zu erkennen, dass die Längsriefen 16 sich bis in den Bereich des Luer-Lock-Außengewindes 5 erstrecken und einen mittels Pfeilen 19 symbolisierten Sterilisationsmittelfluss ermöglichen. Die Längsriefen 16 haben einen radial nach innen gerichteten Durchbruch 20 in den Innenraum 18 des Innenteils 10, wobei der Durchbruch einen Querschnitt nach Art eines Vielecks, insbesondere eines Vierecks, eines Rechtecks oder Quadrats, aufweist. Wie besonders gut in Figur 9 zu erkennen, haben die Längsriefen 16 eine konkave Ausprägung mit einem teilkreisrunden / halbkreisförmigen Querschnitt.

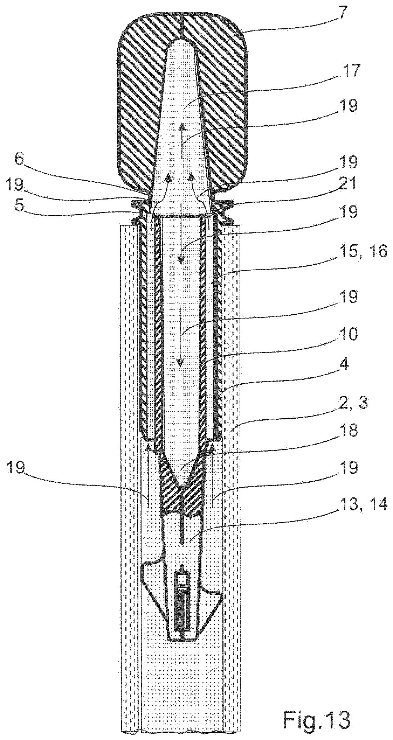

In Figur 13 ist ebenfalls ein Konnektorsystem 1 im Rahmen eines Luer-Lock-Verbinders mit Originalitätsverschluss 7 und Brechkreuz 14 für Schlauchanschlüsse wiedergegeben, wobei auch hier über Pfeile 19 ein Sterilisationsmittelfluss gekennzeichnet ist. Anders als in den Ausführungsbeispielen der Figuren 6 bis 12 sind jedoch keine Durchbrüche 20 vorhanden, sondern Auslässe 21, die direkt mit dem Innenraum 17 des Originalitätsverschlusses 7 korrespondieren.

Die Längsriefen 16 sind nach Art von Kapillarleitungen oder Kapillaren ausgebildet.

## Patentansprüche

1. Konnektorsystem (1) für einen Flüssigkeitsbehälter für pharmazeutische Lösungen, mit einem in ein Fluidleitsystem (2), wie einen Schlauch (3) oder eine Beuteltülle, einsteckbaren Außenteil (4) und mit einem darin angeordneten, vom Außenteil (4) stofflich separaten Innenteil (10), wobei an dem Außenteil (4) ein an einer Sollbruchstelle (6) angebundener und mit Handkraft abreißbarer Originalitätsverschluss (7) vorhanden ist, wobei ferner das Innenteil (10) mit einer Außenseite (11) zumindest abschnittsweise an einer Innenseite (9) des Außenteils (4) anliegt und ein Fluiddurchfluss durch das Innenteil (10) zusätzlich zum Originalitätsverschluss (7) mittels eines am Innenteil (10) angebrachten Verschlusselements (13) verhindert ist, **dadurch gekennzeichnet, dass** an dem Innenteil (10) und dem Außenteil (4) wenigstens ein sich über eine gewisse Länge erstreckendes Fluidleitelement (15), wie eine Rille, eine Riefe, eine Furche oder eine Nut vorhanden ist, wobei das Fluidleitelement (15) derart dimensioniert ist, dass es kapillarartig wirkt, sodass ein Sterilisationsmittel, wie Wasserdampf, zwischen das Innenteil (10) und das Außenteil (4) geleitet werden kann und einen Innenraum (18) im Inneren des Innenteils (10) erreicht, aber das Fluidleitelement (15) so bemessen ist, dass es ein Tropfen ausschließt.

2. Konnektorsystem (1) nach Anspruch 1, **dadurch gekennzeichnet, dass** eine Vielzahl von Fluidleitelementen (15) vorhanden ist.

3. Konnektorsystem (1) nach Anspruch 2, **dadurch gekennzeichnet, dass** die Fluidleitelemente (15) gleichverteilt über den Umfang des Innenteils (10) angeordnet sind und geradlinig oder kurvig verlaufen.

4. Konnektorsystem (1) nach einem der Ansprüche 1 bis 3, **dadurch gekennzeichnet, dass** das Fluidleitelement (15) in die Außenmantelfläche des Innenteils (10) und/oder die Innenmantelfläche des Außenteils (4) eingearbeitet ist.

5. Konnektorsystem (1) nach einem der Ansprüche 1 bis 4, **dadurch gekennzeichnet, dass** das Außenteil (4) eine von einem Zylinder bestimmte Innenmantelfläche aufweist und/oder das Innenteil (10) eine von einem Zylinder bestimmte Außenmantelfläche aufweist.

6. Konnektorsystem (1) nach einem der Ansprüche 1 bis 5, **dadurch gekennzeichnet, dass** das Außenteil (4) auf seiner Außenseite (8) und/oder das Innenteil (10) auf seiner Innenseite (12) nach Art eines Luer-Konus ausgeformt ist.

7. Konnektorsystem (1) nach einem der Ansprüche 1 bis 6, **dadurch gekennzeichnet, dass** auf der dem Originalitätsverschluss (7) zugewandten Seite des Außenteils (4) ein Luer-Lock-Außengewinde (5) vorhanden ist oder ein Luer-Slip-Fortsatz vorhanden ist.

8. Konnektorsystem (1) nach einem der Ansprüche 1 bis 7, **dadurch gekennzeichnet, dass** der Originalitätsverschluss (7) als flügelförmige Handhabe ausgebildet ist und/oder das Verschlusselement (13) als an einer Sollbruchstelle angebundenes Brechkreuz (14) ausgeformt ist.

9. Konnektorsystem (1) nach einem der Ansprüche 1 bis 8, **dadurch gekennzeichnet, dass** das Brechkreuz (14) an einem dem Originalitätsverschluss (7) abgewandten Ende des Innenteils (10) angebunden ist.

10. Medizinisches Versorgungssystem mit einem Beutel wie einem Dialysebeutel, in dem das Konnektorsystem (1) nach einem der Ansprüche 1 bis 9 integriert oder eingesetzt ist.

## Claims

1. A connector system (1) for a fluid container for pharmaceutical solutions, comprising an outer part (4), which is adapted to be inserted into a fluid conducting system (2) such as a hose (3) or a bag spout, and an inner part (10) arranged in said outer part (4) and materially separate therefrom, wherein the outer part (4) has provided thereon a tamper-evident closure (7), which is connected thereto at a predetermined breaking point (6) and which can be torn off by manual force, wherein the inner part (10) further abuts, at least sectionwise, with an external side (11) thereof on an internal side (9) of the outer part (4), and wherein, in addition to the tamper-evident closure (7), a closure element (13) attached to the inner part (10) prevents a flow of fluid through the inner part (10), **characterized in that** the inner part (10) and the outer part (4) have provided thereon at least one fluid conducting element (15), such as a furrow, a flute, a depression or a groove, which extends over a certain length, wherein the fluid conducting element (15) is dimensioned such that it has a capillary effect, so that a sterilizing agent, such as steam, can also be conducted between the inner part (10) and the outer part (4) and reaches an internal space (18) inside the inner part (10), but the fluid conducting element (15) being dimensioned such that dripping is prevented.

2. The connector system (1) according to claim 1, **characterized in that** a plurality of fluid conducting elements (15) is provided.

3. The connector system (1) according to claim 2, **characterized in that** the fluid conducting elements (15) are uniformly distributed over the circumference of the inner part (10) and extend straight or in a curved shape.

4. The connector system (1) according to one of the claims 1 to 3, **characterized in that** the fluid conducting element (15) is formed in the outer circumferential surface of the inner part (10) and/or in the inner circumferential surface of the outer part (4).

5. The connector system (1) according to one of the claims 1 to 4, **characterized in that** the outer part (4) has an inner circumferential surface determined by a cylinder and/or the inner part (10) has an outer circumferential surface determined by a cylinder.

6. The connector system (1) according to one of the claims 1 to 5, **characterized in that** the outer part (4) is configured like a luer cone on the external side (8) thereof and/or the inner part (10) is configured like a luer cone on the internal side (12) thereof.

7. The connector system (1) according to one of the claims 1 to 6, **characterized in that,** on the side of the outer part (4) facing the tamper-evident closure (7), a luer lock external thread (5) or a luer slip extension is provided.

8. The connector system (1) according to one of the claims 1 to 7, **characterized in that** the tamper-evident closure (7) is configured like a wing-shaped handle and/or the closure element (13) is configured as a frangible cross member (14) connected to a predetermined breaking point.

9. The connector system (1) according to one of the claims 1 to 8, **characterized in that** the frangible cross member (14) is connected to the inner part (10) at an inner part end facing away from the tamper-evident closure (7).

10. A medical supply system, comprising a bag, such as a dialysis bag, having the connector system (1) according to one of the claims 1 to 9 integrated or inserted therein.

## Revendications

1. Système connecteur (1) pour un contenant de liquide pour des solutions pharmaceutiques, avec une partie extérieure (4) pouvant être emboîtée dans un système d'acheminement de fluide (2), tel qu'un tuyau flexible (3) ou un embout de poche, et avec une partie intérieure (10) disposée dans celle-ci, séparée matériellement de la partie extérieure (4), dans lequel est présent au niveau de la partie extérieure (4) un système de fermeture inviolable (7) raccordé à un point de rupture théorique (6) et pouvant être déchiré à la main, dans lequel en outre la partie intérieure (10) repose par un côté extérieur (11) au moins par endroits au niveau d'un côté intérieur (9) de la partie extérieure (4) et un passage de fluide à travers la partie intérieure (10) est empêché en plus du système de fermeture inviolable (7) au moyen d'un élément de fermeture (13) installé au niveau de la partie intérieure (10), **caractérisé en ce qu'**au moins un élément d'acheminement de fluide (15) s'étendant sur une certaine longueur, tel qu'une cannelure, une strie, un sillon ou une rainure, est présent au niveau de la partie intérieure (10) et de la partie extérieure (4), dans lequel l'élément d'acheminement de fluide (15) présente des dimensions telles qu'il agit à la manière d'un capillaire de sorte qu'un agent de stérilisation, tel que de la vapeur d'eau, puisse être acheminé entre la partie intérieure (10) et la partie extérieure (4) et atteigne un espace intérieur (18) à l'intérieur de la partie intérieure (10), toutefois l'élément d'acheminement de fluide (15) présente des dimensions telles qu'il exclut une goutte.

2. Système connecteur (1) selon la revendication 1, **caractérisé en ce qu'**une pluralité d'éléments d'acheminement de fluide (15) est présente.

3. Système connecteur (1) selon la revendication 2, **caractérisé en ce que** les éléments d'acheminement de fluide (15) sont disposés selon une répartition homogène sur la périphérie de la partie intérieure (10) et sont rectilignes ou curvilignes.

4. Système connecteur (1) selon l'une quelconque des revendications 1 à 3, **caractérisé en ce que** l'élément d'acheminement de fluide (15) est pratiqué dans la surface enveloppante extérieure de la partie intérieure (10) et/ou dans la surface enveloppante intérieure de la partie extérieure (4).

5. Système connecteur (1) selon l'une quelconque des revendications 1 à 4, **caractérisé en ce que** la partie extérieure (4) présente une surface enveloppante intérieure définie par un cylindre et/ou la partie intérieure (10) présente une surface enveloppante extérieure définie par un cylindre.

6. Système connecteur (1) selon l'une quelconque des revendications 1 à 5, **caractérisé en ce que** la partie extérieure (4) est formée sur son côté extérieur (8) et/ou la partie intérieure (10) est formée sur son côté intérieur (12) à la manière d'un cône de Luer.

7. Système connecteur (1) selon l'une quelconque des revendications 1 à 6, **caractérisé en ce qu'**est présent un filetage extérieur Luer-Lock (5) ou est présent un prolongement Luer-Slip sur le côté, tourné vers le système de fermeture inviolable (7), de la partie extérieure (4).

8. Système connecteur (1) selon l'une quelconque des revendications 1 à 7, **caractérisé en ce que** le système de fermeture inviolable (7) est réalisé sous la forme d'une poignée présentant une forme d'aile et/ou l'élément de fermeture (13) est formé sous la forme d'une croix de rupture (14) raccordée à un point de rupture théorique.

9. Système connecteur (1) selon l'une quelconque des revendications 1 à 8, **caractérisé en ce que** la croix de rupture (14) est raccordée à une extrémité, opposée au système de fermeture inviolable (7), de la partie intérieure (10).

10. Système médical d'alimentation avec une poche, telle qu'une poche de dialyse, dans laquelle le système connecteur (1) selon l'une quelconque des revendications 1 à 9 est intégré ou inséré.
